# EUROPEAN PATENT APPLICATION

(11) **EP 0 629 404 A1**
(43) Date of publication of application: **21.12.1994**
(21) Application number: 94109216.5
(22) Date of filing: 15.06.1994
(51) Int. Cl.: A61K 31/565, A61K 9/14, A61K 9/20, A61K 9/16

(54) **Pharmaceutical composition containing cefditoren pivoxil**

(30) Priority: 16.06.1993 JP 144585/93
(71) Applicant: MEIJI SEIKA KAISHA LTD., Chuo-ku Tokyo 104 (JP)
(72) Inventor: Kikkoji, Toshihiro, c/o MEIJI SEIKA KAISHA, LTD., Yokohama-shi, Kanagawa (JP); Ishizawa, Takayuki, c/o MEIJI SEIKA KAISHA, LTD., Yokohama-shi, Kanagawa (JP); Hayata, Chikako, c/o MEIJI SEIKA KAISHA, LTD., Yokohama-shi, Kanagawa (JP); Ota, Masato, c/o MEIJI SEIKA KAISHA, LTD., Yokohama-shi, Kanagawa (JP)
(74) Representative: WILHELMS, KILIAN & PARTNER Patentanwälte

(57) **Abstract**

This invention provides a pharmaceutical composition having improved oral absorption ability and reduced bitterness comprising (-)-(6R,7R)-2,2-dimethylpropionyloxymethyl 7-[(Z)-2-(2-amino-thiazol-4-yl)-2-methoxyiminoacetamido]-3-[(Z)-2-(4-methylthiazol-5-yl)ethenyl]-8-oxo-5-thia-1-azabicyclo[4.2.0]oct-2-ene-2-carboxylate (to be referred to as "cefditoren pivoxil" hereinafter).

The combination use of cefditoren pivoxil and hydroxypropylcellulose makes it possible to produce the pharmaceutical composition comprising cefditoren pivoxil as an active ingredient, having excellent oral absorption ability and reduced bitterness. Hydroxypropylcellulose is blended with cefditoren pivoxil in a weight ratio of 0.4 or more, preferably 0.8 to 4.

## Description

### FIELD OF THE INVENTION

This invention relates to a pharmaceutical composition for oral administration use having excellent oral absorption ability and less bitterness, which comprises cefditoren pivoxil and hydroxypropylcellulose.

### BACKGROUND OF THE INVENTION

Cefditoren pivoxil is a novel prodrug, (-)-(6R,7R)-2,2-dimethylpropionyloxymethyl 7-[(Z)-2-(2-amino-thiazol-4-yl)-2-methoxyiminoacetamido]-3-[(Z)-2-(4-methylthiazol-5-yl)ethenyl]-8-oxo-5-thia-1-azabicyclo[4.2.0]oct-2-ene-2-carboxylate, which has been prepared by ester bonding of a (pivaloyl)oxymethyl group to the 4-position carboxylic acid of an antibiotic, (+)-(6R,7R)-7-[2-(2-amino-4-thiazolyl)-glyoxyamido]-3-[(Z)-2-(4-methyl-5-thiazolyl)vinyl]-8-oxo-5-thia-1-azabicyclo[4.2.0]oct-2-ene-2-carboxylic acid, 7²-(Z)-(O-methyloxime) (to be referred to as "cefditoren" hereinafter), having broad antibacterial spectrum with the aim of providing the antibiotic with oral absorption ability [K. Sakagami et al., J. Antibiot., 43, 1047-1050 (1990)].

It is considered in general that an increase in the fat-solubility of a drug by esterification leads to acceleration of its membrane permeability in gastrointestinal tracts and results in improvement of its absorption ability from the gastrointestinal tracts. However, absorption ability of drugs is closely related to their solubility and an increase in fat-solubility due to esterification reduces properties such as water wettability, dispersibility, solubility and the like. Thus, there is a serious drawback in that satisfactory improvement in the absorption ability cannot always be attained by such a means. In order to overcome such a problem, attempts have been made in recent years to improve absorption ability of drugs using solubilizing agents such as organic acids, cyclodextrin and the like [T. Kikkoji et al., Yakuzaigaku, 52, 120-127 (1992) and JP-A-1-268637; the term "JP-A" as used herein means an unexamined published Japanese patent application"]. However, when an organic acid is used, its strong acidity causes difficulty in taking drugs. The use of cyclodextrin also causes another serious problem in terms of drug-taking, because it forms a water soluble complex with cefditoren pivoxil and the complex formation entails a sharp increase in the solubility of the drug itself and hence considerable amplification of the drug-inherent bitterness.

It is possible to a certain degree to solve such problems by applying film coating to the surface of tablets and granules. At the time of actual clinical or domestic drug-taking, however, pharmaceutical preparations are generally administered by pulverizing them or suspending them in water like dry syrups so that the aged and children can take them easily. In consequence, masking of tastes by coating is not sufficient as a substantial measure of solving these problems in view of practical use.

### SUMMARY OF THE INVENTION

The inventors of the present invention have conducted intensive studies and found that a pharmaceutical composition which comprises cefditoren pivoxil and a water soluble high polymer, hydroxypropylcellulose, shows improved water wettability and dispersibility, as well as dissolution of the drug from the composition, without affecting the solubility of the drug itself and, as the result, it provides markedly improved absorption ability of the drug without increasing its bitterness.

The present invention provides a pharmaceutical composition which comprises cefditoren pivoxil and hydroxypropylcellulose.

### BRIEF DESCRIPTION OF THE DRAWINGS

Fig. 1 is a graph showing elution curve (average, n = 3) of each of the present and comparative compositions. In Fig. 1, ■ stands for Example 3, x for Example 5, ● for Example 6, ◇ for Reference Example 1 and △ for Reference Example 2.

Fig. 2 shows a relationship between the average particle size of the present composition suspended in water and the hydroxypropylcellulose/cefditoren pivoxil mixing ratio.

Fig. 3 shows changes in a concentration of cefditoren in plasma (average, n = 8) after oral administration of each of the present and comparative compositions to Beagle dogs. In Fig. 3, ● stands for Example 6, ■ for Reference Example 2 and x for Reference Example 3.

### DETAILED DESCRIPTION OF THE INVENTION

The pharmaceutical composition of the present invention can be produced by mixing 0.4 or more weight part, preferably 0.8 to 4 weight parts, more preferably 1 to 2 weight parts, of hydroxypropylcellulose with one weight part of cefditoren pivoxil. If the mixing ratio of hydroxypropylcellulose to cefditoren pivoxil is smaller than 0.4 by weight ratio, desired dispersibility and dissolution ability cannot be obtained. On the other hand, though not particularly restricted, the upper limit of the mixing ratio is preferably 4 or less by weight ratio, because the ratio exceeding 4 would make the resulting preparation bulky and hence entail some problems such as difficulty in taking the preparation. Several types of hydroxypropylcellulose having different viscosities can be used in the present invention. The types of hydroxypropylcellulose include type H having viscosity of 1,000 to 4,000 cps, type M of 150 to 400 cps, type L of 6.0 to 10.0 cps, type SL of 3.0 to 5.9 cps and type SSL of 2.0 to 2.9 cps, when viscosity is measured in a 2% aqueous solution at 20°C. Medium to low viscosity types (types M, L, SL and SSL) are preferred to a high viscosity type (type H). The type L is particularly preferred.

For the purpose of improving disintegration ability and dispersibility, the composition of the present invention may be mixed further with 5 to 70% by weight, based on the weight of the preparation, of disintegrating agents such as carboxymethylstarch sodium, crospovidone, croscarmellose sodium, carboxymethylcellulose calcium, low substitution hydroxypropylcellulose and the like.

The composition may also be mixed with pharmaceutically acceptable binders, fillers, sweeteners, perfumes, lubricants and the like, if required.

The pharmaceutical composition of the present invention for oral administration use may be made into various dosage forms such as capsules, granules, powders, tablets, dry syrups and the like with no particular limitation. These solid preparations can be produced by wet granulation or solvent drying method. For example, in the wet granulation method, an appropriate amount of water is added to a mixture of cefditoren pivoxil and hydroxypropylcellulose, the resulting mixture is subjected to kneading and granulation and then the thus obtained granules are made into various dosage forms. In the case of the solvent drying method, a mixture of cefditoren pivoxil and hydroxypropylcellulose is dissolved in an organic solvent such as ethanol or dichloromethane or a mixed solvent thereof with or without containing water, the resulting solution is dried by spray drying, vacuum drying or the like means and then the thus dried material is made into various dosage forms.

Cefditoren pivoxil is contained in the pharmaceutical composition of the present invention in an amount of 50 to 200 mg potency/tablet or capsule and 5 to 20 wt% (potency) in the case of granules, powders or dry syrups, provided that 100 mg potency corresponds to about 130 mg weight.

The pharmaceutical composition of the present invention can be administered to a patient suffering from infectious diseases, such as folliculitis, mastitis, tonsilitis and the like, in a dose of 100 to 200 mg potency per adult and 3 mg potency/kg per child, in terms of the active ingredient, two or three times per day, though the dosage varies depending on patient's age, symptom and the like.

The following examples are provided to further illustrate the present invention. It is to be understood, however, that the examples are for purpose of illustration only and are not to be construed to limit the scope of the present invention.

### EXAMPLE 1

A uniform powder mixture composed of 130 g of cefditoren pivoxil and 260 g of hydroxypropylcellulose (type L) was dissolved in 3 kg of dichloromethane. Thereafter, the solvent was removed using an evaporator and the resulting residue was pulverized and passed through a screen to obtain powders having the following composition per 390 mg.

| | |
|---|---|
| cefditoren pivoxil | 130 mg |
| hydroxypropylcellulose (type L) | 260 mg |
| total | 390 mg |

### EXAMPLE 2

In accordance with the method described in Example 1, powders having the following composition per 390 mg were obtained.

| | |
|---|---|
| cefditoren pivoxil | 130 mg |
| hydroxypropylcellulose (type SL) | 260 mg |
| total | 390 mg |

### EXAMPLE 3

A uniform powder mixture composed of 130 g of cefditoren pivoxil and 65 g of hydroxypropylcellulose (type L) was dissolved in 3 kg of dichloromethane, and 305 g of D-mannitol was suspended in the resulting solution. Thereafter, the solvent was removed using an evaporator and the resulting residue was pulverized and passed through a screen to obtain granules having the following composition per 500 mg.

| | |
|---|---|
| cefditoren pivoxil | 130 mg |
| hydroxypropylcellulose (type L) | 65 mg |
| D-mannitol | 305 mg |
| total | 500 mg |

### EXAMPLE 4

In accordance with the method described in Example 3, granules having the following composition per 1,000 mg were obtained.

| | |
|---|---|
| cefditoren pivoxil | 130 mg |
| hydroxypropylcellulose (type L) | 390 mg |
| D-mannitol | 480 mg |
| total | 1,000 mg |

### EXAMPLE 5

A uniform powder mixture composed of 130 g of cefditoren pivoxil and 260 g of hydroxypropylcellulose (type L) was dissolved in 3 kg of dichloromethane, the resulting solution was dried to a powder by spray drying and the powder was subsequently filtered through a screen. Thereafter, the thus prepared powder was mixed with 130 g of croscarmellose sodium and 3 g of magnesium stearate, and the resulting mixture was encapsulated to obtain capsules each having the following composition.

| | |
|---|---|
| cefditoren pivoxil | 130 mg |
| hydroxypropylcellulose (type L) | 260 mg |
| croscarmellose sodium | 130 mg |
| magnesium stearate | 3 mg |
| total | 523 mg |

### EXAMPLE 6

A uniform powder mixture composed of 130 g of cefditoren pivoxil and 130 g of hydroxypropylcellulose (type L) was kneaded with an appropriate amount of water and made into granules. Thereafter, the thus prepared granules were mixed with 130 g of carboxymethylstarch sodium and 3 g of magnesium stearate, and the resulting mixture was subjected to tablet making in the usual manner to obtain tablets each having the following composition.

| | |
|---|---|
| cefditoren pivoxil | 130 mg |
| hydroxypropylcellulose (type L) | 130 mg |
| carboxymethylstarch sodium | 130 mg |
| magnesium stearate | 3 mg |
| total | 393 mg |

### REFERENCE EXAMPLE 1

Using a uniform powder mixture composed of 130 g of cefditoren pivoxil and 130 g of hydroxypropylmethyl cellulose, tablets each having the following composition were produced in accordance with the method described in Example 6.

| | |
|---|---|
| cefditoren pivoxil | 130 mg |
| hydroxypropylmethylcellulose | 130 mg |
| carboxymethylstarch sodium | 130 mg |
| magnesium stearate | 3 mg |
| total | 393 mg |

### REFERENCE EXAMPLE 2

Using 2% hydroxypropylmethylcellulose aqueous solution as a binding solution, 130 g of cefditoren pivoxil was subjected to wet granulation. Thereafter, the thus prepared granules were mixed with 130 g of carboxymethyl-starch sodium and 3 g of magnesium stearate, and the resulting mixture was formulated into tablets in the usual manner to obtain tablets each having the following composition.

| | |
|---|---|
| cefditoren pivoxil | 130 mg |
| hydroxypropylmethylcellulose | 7 mg |
| carboxymethylstarch sodium | 130 mg |
| magnesium stearate | 3 mg |
| total | 270 mg |

### REFERENCE EXAMPLE 3

Using a uniform powder mixture composed of 130 g of cefditoren pivoxil and 260 g of β-cyclodextrin, tablets each having the following composition were produced in accordance with the method described in Reference Example 2.

| | |
|---|---|
| cefditoren pivoxil | 130 mg |
| β-cyclodextrin | 260 mg |
| hydroxypropylmethylcellulose | 7 mg |
| carboxymethylstarch sodium | 130 mg |
| magnesium stearate | 3 mg |
| total | 530 mg |

The following test examples are provided to illustrate effects of the present invention further in detail.

### TEST EXAMPLE 1

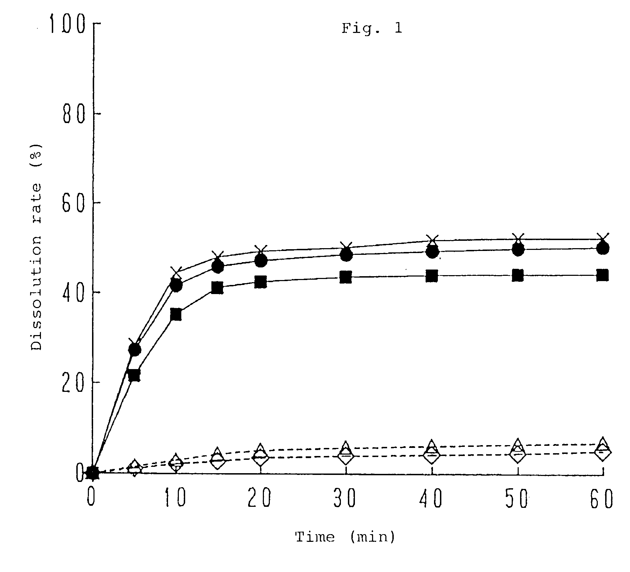

The pharmaceutical preparations obtained in Examples 3, 5 and 6 and Reference Examples 1 and 2 were examined for their dissolution abilities in accordance with the second method (paddle method, 50 rpm) of The Pharmacopoeia of Japan using 900 ml of water (37 ± 0.5°C) as the test solution. The results are shown in Fig. 1. As shown in Fig. 1, the pharmaceutical composition of the present invention shows markedly improved dissolution ability.

### TEST EXAMPLE 2

A total of 20 powder preparations having different mixing ratios of cefditoren pivoxil and type L hydroxypropylcellulose (hydroxypropylcellulose/cefditoren pivoxil ratio; 0.2, 0.4, 0.6, 0.8, 1.0, 1.2, 1.4, 1.6, 1.8, 2.0, 2.2, 2.4, 2.6, 2.8, 3.0, 3.2, 3.4, 3.6, 3.8 and 4.0) were prepared in accordance with the method described in Example 1, and a predetermined amount of each of the thus prepared powders was suspended in water to measure average size of the suspended particles using a particle size distribution analyzer (Submicron Particle Size Analyzer NICOMP Model 370, manufactured by Nozaki Sangyo).

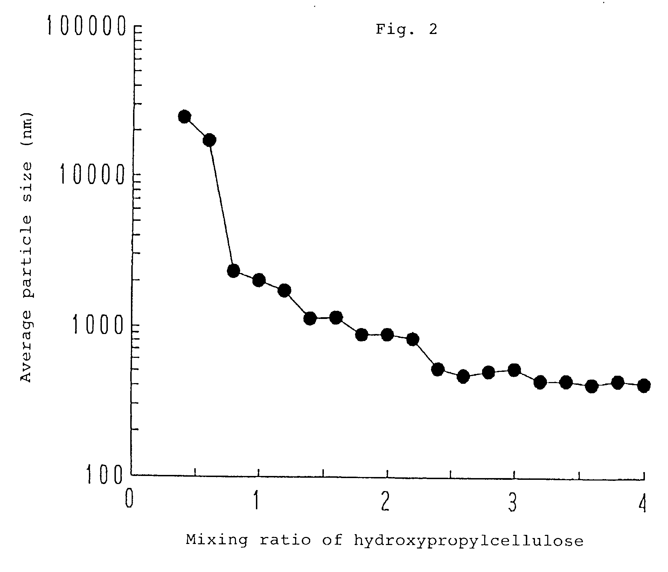

The results are shown in Fig. 2. When the mixing ratio of hydroxypropylcellulose was smaller than 0.4, average particle size cannot be measured because it exceeded 50 µm. On the other hand, the average particle size became small as the amount of hydroxypropylcellulose increased with an asyndetic value of about 400 nm. Thus, it was confirmed that the particle size of powders suspended in water becomes markedly small when hydroxypropylcellulose is mixed, hence resulting in the considerable improvement of the dissolution ability due to improved dispersibility and increased surface area of the particles.

### TEST EXAMPLE 3

Using various types of hydroxypropylcellulose having different viscosities, powder preparations having a cefditoren pivoxil/hydroxypropylcellulose mixing ratio of 1:2 were prepared in accordance with the method described in Example 1, and their average suspension particle sizes were measured in the same manner as described in Test Example 2. The results are shown in Table 1. The average particle size became minimum when type L hydroxypropylcellulose was used.

**Table 1**

| Average size of particles suspended in water | |
|---|---|
| Hydroxypropylcellulose | Average particle size (nm) |
| type H | 49304 |
| type M | 30885 |
| type L | 642 |
| type SL | 1005 |
| type SSL | 3051 |

### TEST EXAMPLE 4

In order to confirm effects of the present invention more clearly, absorption ability of the drug when orally administered to Beagle dogs was evaluated.

Each of the pharmaceutical preparations obtained in Example 6 and Reference Examples 2 and 3 was orally administered to female Beagle dogs weighing about 10 kg which had been allowed to fast for 16 hours, in a dose of 260 mg (two tablets) as cefditoren pivoxil per animal together with 30 ml of water. After 0.25, 0.5, 1, 2, 4, 6 and 8 hours of the administration, a concentration of cefditoren in plasma was measured by a high performance liquid chromatography.

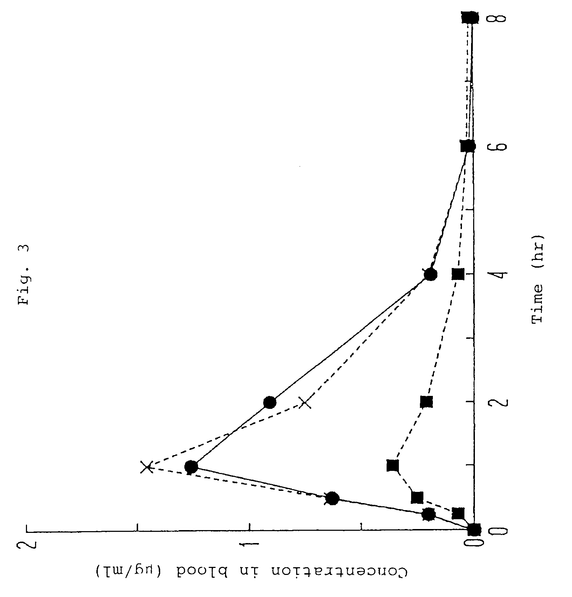

The results are shown in Fig. 3. In comparison with the preparation of Reference Example 2 composed of a standard formulation, the preparation of the present invention in which hydroxypropylcellulose had been blended showed distinctive increase in the absorption ability which was comparable to the level of the preparation of Reference Example 3 that had been produced based on known absorption enhancing techniques.

### TEST EXAMPLE 5

The tablets which had been produced in Example 6 and Reference Example 3 and whose improved absorption abilities have been confirmed in Test Example 4 were vulcanized, and the degree of bitterness of the resulting powders was evaluated by each panelist who held a one tablet-equivalent portion of each powdered sample in the mouth for 10 seconds together with 30 ml of water, spat out the whole contents and then judged the degree of bitterness as "slightly bitter" (one point), "bitter" (two points) or "very bitter" (three points). In this instance, 10 panelists were evenly divided into two groups, and the tablet of Example 6 was evaluated by the first group, and the tablet of Reference Example 3 by the second group. Thereafter, the remaining contents in the mouth were thoroughly rinsed out with a large volume of water to repeat evaluation of the tablets by alternating the two groups 30 minutes after the rinsing.

The results are shown in Table 2. As is evident from the results shown in Table 2, bitterness of the pharmaceutical composition of the present invention is significantly smaller than that of the preparation of Reference Example 3.

**Table 2**

| Evaluation of bitterness | | |
|---|---|---|
| Panelist No. | Inventive Example 6 | Reference Example 3 |
| First group | | |
| 1 | 1 | 3 |
| 2 | 1 | 2 |
| 3 | 1 | 2 |
| 4 | 1 | 3 |
| 5 | 2 | 3 |

| Second group | | |
|---|---|---|
| 6 | 1 | 2 |
| 7 | 2 | 3 |
| 8 | 1 | 2 |
| 9 | 1 | 2 |
| 10 | 1 | 3 |
| Average value ± SD | 1.2 ± 0.4 * | 2.6 ± 0.5 |

| | | |
|---|---|---|
| *: significant with a level of significance of 5% | | |

### TEST EXAMPLE 6

A 130 mg portion of cefditoren pivoxil or one tablet of the tablets obtained in Example 6 or Reference Example 3 was added to 15 ml of water followed by ultrasonication for 10 minutes for dispersion and then subjected to centrifugation to measure a concentration of cefditoren pivoxil in the resulting supernatant fluid by a liquid chromatography.

The results are shown in Table 3. Solubility of the drug in the pharmaceutical composition of the present invention was almost the same as that of the drug itself, but the drug in the preparation of Reference Example 3 showed a markedly increased solubility. In consequence, it was revealed that the smaller bitterness of the pharmaceutical composition of the present invention than the preparation of Reference Example 3 as confirmed in Test Example 5 was based on such a difference in the drug solubility.

**Table 3**

| Solubility of cefditoren pivoxil (µg/ml) | |
|---|---|
| Cefditoren pivoxil | Average ± SD (n = 3) |
| Raw powder | 72.3 ± 5.4 |
| Example 6 | 76.1 ± 6.8 |
| Reference Example 3 | 992.8 ± 33.5 |

Thus, the present invention provides an antimicrobial composition for oral administration use having excellent oral absorption ability with less bitterness and high drug dispersion and dissolution abilities in water.

While the invention has been described in detail and with reference to specific examples thereof, it will be apparent to one skilled in the art that various changes and modifications can be made therein without departing from the spirit and scope thereof.

## Claims

1. A pharmaceutical composition which comprises cefditoren pivoxil and hydroxypropylcellulose.

2. The pharmaceutical composition according to claim 1, wherein a mixing ratio of hydroxypropylcellulose to cefditoren pivoxil ranges from 0.4 to 4 by weight.

3. The pharmaceutical composition according to claim 1, wherein a mixing ratio of hydroxypropylcellulose to cefditoren pivoxil ranges from 0.8 to 4 by weight.

4. The pharmaceutical composition according to claim 1, wherein said hydroxypropylcellulose has medium to low viscosity.

5. The pharmaceutical composition according to claim 4, wherein said hydroxypropylcellulose is type L.

6. The use of hydroxypropylcellulose for preparation of a pharmaceutical composition comprising cefditoren pivoxil as an active ingredient.
